# EUROPEAN PATENT APPLICATION

(11) **EP 3 263 165 A1**
(43) Date of publication of application: **03.01.2018**
(21) Application number: 16754793.4
(22) Date of filing: 27.01.2016
(51) Int. Cl.: A61M 16/04

(54) **DEVICE FOR IMMOBILISING OXYGEN-ADMINISTERING TUBES IN ANAESTHETISED PATIENTS**

(30) Priority: 26.02.2015 ES 201530235 U
(71) Applicant: Clip-Fab R&D Medical Instruments, S.L., 08017 Barcelona (ES)
(72) Inventor: FERRANDIZ CATALÁN, Antonio, 03016 Alicante (ES)
(74) Representative: Isern-Jara, Nuria
(86) International application number: PCT/ES2016/000011
(87) International publication number: WO 2016/135355

(57) **Abstract**

The invention relates to a device for immobilizing oxygen administering tubes in anaesthetized patients, where the corresponding endotracheal tube comprises, on the distal end thereof, a balloon or bag to be placed in the branch of the trachea of the patient, characterised in that a pair of balloons or bags are connected to the endotracheal tube itself, one of them having an omega shape, and the other being spherical and larger, and both being arranged inside the mouth of the patient and being associated with respective valves for the inflation thereof.

## Description

### OBJECT OF THE INVENTION

The present invention relates to a device for immobilizing or fastening endotracheal tubes for the administration of oxygen or other gases, for example anesthetics, in patients, the obvious purpose of which is to prevent the classic tube entering the throat of a patients for injecting oxygen, from being immobilized and therefore unable to be displaced during the corresponding medical intervention.

Consequently, the device is intended for application in the health sector.

### BACKGROUND OF THE INVENTION

It is well known that in certain medical and/or surgical interventions on anesthetized patients, said patients receive assisted ventilation through a tube that is introduced through the mouth and in turn connected to a ventilator.

Said tube does not have sufficient securing means to ensure its immobility during the intraoperative period, so that sometimes due to unwanted, involuntary or any other kind of movements it can be accidentally displaced and lead to inadequate assisted ventilation that could cause irreversible brain damage in extreme situations.

In order to try overcoming this problem, the tube is usually immobilized by using tape or bandages, a solution that is not as effective as would be desirable. Furthermore, it is not always possible to apply tape or bandages to the patient's face, for example, in occasions when the operation is precisely being carried out in that area, such as in a facelift of any other type of intervention.

There are certain endotracheal tubes that have a balloon at their distal end intended to be housed in the bifurcation of the trachea, so that after it is inflated with air or serum through the corresponding valve, it becomes an element that ensures immobility of the endotracheal tube with respect to this distal area of the tube.

However, a patient may be subjected to different changes in position during an intervention (Trendelenburg, reverse Trendelenburg, prone position) and said changes in position can bring about displacement of the endotracheal tube in spite of said structuring.

In any case, regardless of whether or not the endotracheal tube incorporates an immobilizing balloon, it is necessary to fasten said tube with tape, bandages or other elements onto the patient's face, with the limitations that have been described above.

### DESCRIPTION OF THE INVENTION

The device that is being recommended has been conceived to solve the problem described above in a simple yet very effective way.

To this end, and more specifically, the device of the invention is based on the fact that in addition to the balloon incorporated by the endotracheal tube that is housed in the bifurcation of the trachea, a double balloon is included, one with an omega configuration and another which is spherical, such that the first one, when inflated, is arranged on the endotracheal tube and places pressure on it, while the second one, just like the former one linked to said tube, establishes a means for immobilizing said endotracheal tube, as a consequence of which both balloons are housed inside the mouth, i.e., they are intraoral and are provided with inflation valves, operation which can be carried out by means of simple syringes.

The device is complemented by discs or washers that function as stops and are located between the gums and the lips in order to avoid displacements of the device.

This way, an easy-to-operate device is achieved, effectively fastening the endotracheal tube, preventing it from moving for as long as the patient remains intubated.

By means of the described device, the area of the face is left free, including the lips and the nose, and this evidently facilitates certain interventions in which said areas form part of the operative field.

Furthermore, by means of the described device, the endotracheal tube can be displaced voluntarily towards the center, left or right, according to the requirements of the moment and the time of the intervention, which is possible due to the omega shape of one of the two intraoral balloons.

It is also worth noting that the device prevents the endotracheal tube from being subjected to accidental displacements that would prevent adequate assisted ventilation, since immobilization is independent from the position adopted by the endotracheal tube.

Another important characteristic worth noting is that the larger internal balloon, which is arranged in the mouth, provides a tamponade of the larynx in order to prevent sucking in gastric or bloody contents that may appear during the intervention.

Finally, it is worth highlighting the fact that since the fastening and immobilizing of the endotracheal tube is carried out by means of intraoral balloons, this allows the disappearance of irritations or cuts and scrapes on the skin of the patient's face and neck, which usually occur with traditional immobilizing systems, especially when it comes to long interventions in which the patient has to be in prone position.

The device may form part of or be incorporated into the endotracheal tube, or it may be incorporated subsequent to the intubation carried out on the patient, taking along a fastening of the device to the endotracheal tube by means of a kind of clip, flange or the like.

### DESCRIPTION OF THE DRAWINGS

As a complement to the description provided below, and for the purpose of helping to make the characteristics of the invention more readily understandable, in accordance with a preferred practical embodiment thereof, said description is accompanied by a set of figures, which by way of illustration and not limitation represent the following:
Figure 1 shows a schematic representation of a device for immobilizing oxygen administration tubes in anesthetized patients carried out in accordance with the object of the invention.
Figure 2 shows a side view of the device applied to an endotracheal tube through which oxygen is being supplied to a patient.

### PREFERRED EMBODIMENT OF THE INVENTION

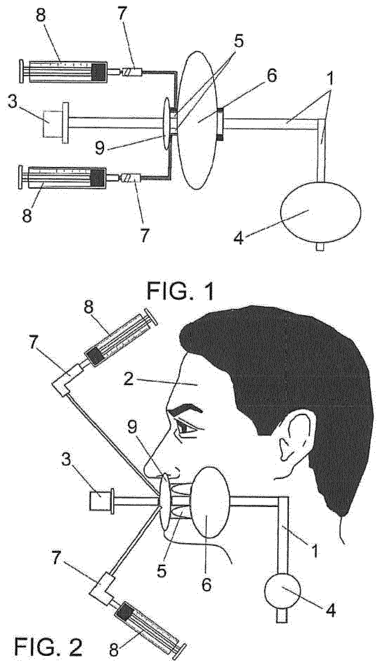

As can be seen in the above-mentioned figures, the device of the invention is intended to be applied to an endotracheal tube (1) by means of which oxygen or another gas is supplied to a patient (2) undergoing a medical or surgical operation, including a corresponding ventilator fastening element (3) and a balloon (4) at its distal end, which is positioned in the bifurcation of the trachea of the patient (2) itself, as shown in Figure 2.

Based on these characteristics, the novelty of the invention consists in that in addition to the balloon (4) two intraoral balloons (5-6) are incorporated on the endotracheal tube (1) i.e., they are arranged inside the mouth of the patient (2), as shown in Figure 2, so that the balloon (5) has an omega configuration and its inflation provides pressure on the endotracheal tube (1), the other larger balloon (6) remaining in an area further back inside the mouth, providing a tamponade of the larynx in order to prevent sucking in gastric or bloody contents that may appear during the corresponding intervention.

By means of this device the endotracheal tube (1) can be displaced voluntarily to one side or the other, or even to the center, avoiding any kind of involuntary displacement, without needing external elements such as tapes or bandages or other fastening elements used conventionally.

Both intraoral balloons (5-6) are inflated by means of the corresponding valves (7) with syringes or similar elements (8) for applying air or any other fluid, such as serum, for said inflation.

Finally, in order to avoid involuntary displacement in the axial direction, i.e., inwards or outwards, a disc-like stop element (9) is provided, which is inserted between the gums and the lips of the patient (2), and which is logically pressure-fitted, like a clamp or similar, onto the endotracheal tube (1) itself.

## Claims

1. A device for immobilizing oxygen administration tubes in anesthetized patients, wherein the corresponding endotracheal tube incorporates, at its distal end, a balloon intended to be located in the bifurcation of the trachea of the patient, **characterized in that** it incorporates a pair of balloons linked to the endotracheal tube itself, one of them having an omega configuration and the other being spherical and larger in size, intended to be located inside the patient's mouth, both elements being related to respective valves for the inflation thereof.

2. The device for immobilizing oxygen administration tubes in anesthetized patients according to claim 1, **characterized in that** the intraoral balloon with an omega configuration is arranged in front of the larger spherical intraoral balloon.

3. The device for immobilizing oxygen administration tubes in anesthetized patients according to claim 1, **characterized in that** it is complemented with a disk-like element, that can be fastened to the tube, intended to be arranged between the lips and gums of the patient.

4. The device for immobilizing oxygen administration tubes in anesthetized patients, according to the preceding claims, **characterized in that** the set of intraoral balloons as well as the disk-like element for stabilizing the tube are workable with respect to said tube.
